Europäisches Patentamt

European Patent Office

Office européen des brevets

(11) Publication number : **0 512 769 A2**

# (12) EUROPEAN PATENT APPLICATION

(21) Application number : **92303973.9**

(22) Date of filing : **01.05.92**

(51) Int. Cl.$^5$ : **A61L 27/00, A61F 2/06, C12N 5/00, C12M 3/00**

(30) Priority : **03.05.91 US 695474**

(43) Date of publication of application : **11.11.92 Bulletin 92/46**

(84) Designated Contracting States : **DE FR GB IT**

(71) Applicant : **BECTON, DICKINSON & COMPANY**
**One Becton Drive**
**Franklin Lakes New Jersey 07417-1880 (US)**

(72) Inventor : **Alchas, Paul G.**
**29 Ponds Circle**
**Wayne, NJ 07470 (US)**
Inventor : **Di Pisa, Joseph A.**
**84 Maryann Lane**
**Wyckoff, NJ 07481 (US)**

(74) Representative : **Ruffles, Graham Keith et al**
**MARKS & CLERK 57-60 Lincoln's Inn Fields**
**London WC2A 3LS (GB)**

(54) **Device and method for collecting and processing fat tissue and procuring microvessel endothelial cells.**

(57) A device is provided, and a method, for collecting and processing tissue to produce an endothelial cell product having a vessel for rinsing, draining, digesting and isolating tissue. The vessel has a rinsing and digesting chamber for containing tissue during processing. An inlet in the rinsing and digesting chamber allows entry of rinsing solution and tissue from liposuction means. A drain chamber is in the vessel and is in fluid communication with the rinsing and digesting chamber. The drain chamber connects with a vacuum source. A screen in the vessel separates the rinsing and digesting chamber from the drain chamber to allow filtered transfer. An isolation chamber in the vessel is separated by another screen. A channel in fluid communication with the isolation chamber includes a pair of ports controlled by valve means to be selectively in fluid communication with the channel. After processing, the channel has a pellet of microvessel endothelial cells and the valve means may place the pellet of microvessel endothelial cells in fluid communication with the ports. The method includes providing the vessel, introducing tissue to be processed, orienting the vessel to screen the tissue, introducing an enzyme and agitating to digest the tissue, centrifuging the vessel to transfer the cells from the digested tissue, and isolating the cells for retrieval.

EP 0 512 769 A2

While an autologous vein remains the graft of choice, advanced vascular disease and prior surgical intervention limit the availability of autologous grafts. Therefore, the use of synthetic grafts provides a means for restoring blood flow to ischemic areas when no alternative is available. Over the past three decades, artificial grafts have been used to provide immediate restoration of blood flow to areas of ischemia as a result of atherosclerotic vascular disease. In addition, they have been used to provide vascular access for hemodialysis in patients with chronic renal failure, and in the repair of arterial aneurysms.

Although initially successful in restoring perfusion to ischemic tissues, the long term prognosis for these grafts is not encouraging. Commercially available grafts are far from ideal due to their inherent thrombogenicity. Over an extended period of time, grafts less than 4 mm in diameter lose their patency as they become occluded via fibrin deposition and cellular adhesion. This process appears to be secondary, and to be due in part to the thrombogenic nature of the nude, i.e. nonendothelialized, surface of an implanted prosthesis. See Berger et al., "Healing of Arterial Prostheses in Man: It's Incompleteness", Ann. Surg. 175:118-27 (1972).

Thus, much current research is being focused on lining prostheses with human endothelial cells, for producing a non-thrombogenic endothelial cell surface such as exists in native human vessels. In dogs, seeding of endothelial cells onto both small and large diameter grafts has been shown to result in a complete endothelial cell lining in one to four months. Since vascular endothelium is said to represent a unique non-thrombogenic surface, endothelial cells are reported to be "the first logical choice for lining small diameter vascular grafts." The transplantation of a functional endothelial cell lining onto the surface of a vascular graft has proven to increase patency rates and decrease thrombus formation on the flow surface in animal models.

Past and present studies have focused on the isolation of large vessel endothelial cells from vein segments, with the subsequent seeding of these cells on the graft lumenal surface. Tissue culture advances have also made the generation of large numbers of endothelial cells for high-density seeding on vascular prosthesis possible. These techniques have major drawbacks in the clinical setting. Endothelialization occurs at a slow rate, when low density seeding techniques are applied. High-density seeding, using cultured endothelial cells, requires the use of undefined media not easily applicable to the clinical setting.

It has been recognized that human microvascular endothelial cells i.e. the cells which are derived from capillaries, arterioles, and venules, will function suitably in place of large vessel cells even though there are morphological and functional differences between large vessel endothelial cells and microvessel endothelial cells in their native tissues. Microvascular endothelial cells are present in an abundant supply in body tissue, most notably in fat tissue, and may be used to establish a degree of preimplantation confluence, i.e. at least 50%, which dramatically improves the prognosis of most implants. For purposes of further description, fat tissue is designated as the exemplary source of microvascular endothelial cells, but it is recognized that endothelial cells from other tissues may be used as well.

To overcome the problems associated with seeding large vessel endothelial cells on prosthetic grafts, methods were developed for the isolation of microvessel endothelial cells from autologous adipose tissue, followed by high density seeding of a vascular prosthesis.

Although microvessel endothelial cells have been shown to be capable of endothelializing a blood-contacting surface, methods of procuring and depositing these cells in an operating room setting present special considerations. A vascular graft or other implant is treated to confluence using microvascular endothelial cells which are separated from fat, which is obtained at the beginning of the same uninterrupted surgical procedure. Fat tissue is removed from the patient after sterile conditions have been established. Microvascular endothelial cells in that fat area are then quickly separated from their related tissue by enzymatic digestion and centrifugation, and are used to treat a surface which is then implanted in the patient during the latter stages of the same operation. This procedure permits a patient to receive a graft which has been treated up to or above confluence with his own fresh endothelial cells.

The microvascular rich tissue obtained is perinephric fat, subcutaneous fat, omentum, or fat associated with the thoracic or peritoneal cavity. This tissue is then subjected to digestion using a proteolytic enzyme such as collagenase, comprising caseanase and trypsin, which is incubated with the tissue until the tissue mass disperses to produce a tissue digest. The microvascular endothelial cells are then separated from the digest using low speed centrifugation to produce an endothelial cell rich pellet. The pellet is washed with a buffered saline solution.

The resulting microvascular endothelial cells are then, preferably, suspended in buffered saline solution containing plasma protein, preferably about 1% plasma protein. This suspension, which comprises, on a volumetric basis, a pellet to solution ratio of 1:5 to 1:15, or preferably about 1:10, is then used to treat the surface by incubating cells with that surface until sufficient adherence of the microvascular endothelial cells to that surface occurs to provide at least 50% confluence. As a result, an improved graft implant is provided having endothelialized surfaces which are either confluent, or which reach confluence quite rapidly (within one population doubling) following implantation.

Implants which can be treated to produce such an endothelial cell lining include but are not limited to, for example, intravascular devices such as artificial vascular prostheses, artificial hearts, and heart valves. The kit and methods of this invention for endothelializing surfaces can be used for surfaces comprised of known synthetic materials such as polyester, polytetrafluoroethylene, or naturally occurring materials, such as umbilical vein, saphenous vein, and native bovine artery.

Methods currently used employ standard laboratory equipment such as beakers, flasks, centrifuge tubes, shaker baths, pipettes, syringes, and sterile hoods. In one method the donated tissue is immediately transferred to ice cold buffered saline (pH 7.4) wherein the buffering agent is preferably a phosphate, i.e. a phosphate buffered saline (PBS). The tissue is minced with fine scissors and the buffer decanted. The proteolytic enzyme collagenase, containing caseanase and trypsin, is added to the tissue and incubated at 37 degrees C until the tissue mass disperses. The digestion occurs within 30 minutes and generally should be less than 20 minutes. The digest is transferred to a sterile test tube and centrifuged at low speed (700 x g) in a table top centrifuge for 5 minutes at room temperature.

The pellet of cells thus formed consists of greater than 95% endothelial cells. These endothelial cells are described herein as microvascular endothelial cells (MED) since they originate from the arterioles, capillaries and venules, all elements of the microvasculature. The MEC pellet is washed 1 time by centrifugation with buffered saline, preferably PBS. The MEC suspension is then, preferably, pelletized by centrifugation (200 x g) and the pellet resuspended with protein containing buffer solution. This resuspension should be performed at a ratio of approximately 1:5 to 1:15 or about 1:10 volumes of packed microvascular endothelial cells to buffer solution. The cell suspension is added to tubular grafts and the ends clamped, or the cells layered upon the surface to be treated.

Optimum periods for cell interaction vary upon the material of the prosthesis, the nature of any pretreatments it may have received, and whether the surface of the prosthesis has been modified to improve its acceptance of the MEC. Following incubation for a sufficient time to permit adherence of the endothelial cells with the prosthesis surface, the surface is washed with a protein containing buffer.

In United States Patent No. 4,820,626, and related applications, methods of treating a graft surface with endothelial cells are disclosed. According to those methods, subcutaneous adipose tissue is aspirated via a cannula and transferred by vacuum into a mucous trap. The trap is then transferred to a sieve inside a funnel which is placed in a sterile beaker. A rinsing solution is then poured over the tissue to remove red blood cells and lysed fat. The tissue is manually poured into a sterile Erlermeyer flask containing collagenase solution and agitated at 37°C for 20 minutes. The collagenase slurry is manually poured into sterile conical centrifuge tubes, and spun for seven minutes at 700 x g. The endothelial cells are then pipetted out of the tube. A graft is tied to a male luer extension, and secured within a tube. The cells are resuspended in serum protein media and drawn into a syringe using a needle and syringe. The cells are forced into the lumen of the graft. The graft is manually rotated for 2 hours.

In spite of these advances, a need still exists for a simple, reliable method for producing endothelial cell coatings on a graft in an operating room setting ,and this invention solves that need. The present invention provides for the ready isolation of large quantities of endothelial cells which can be easily performed in an operating room. While endothelial cells can be isolated from tissues other than fat, such as brain, lung, retina, adrenal glands, liver and muscle, the use of fat tissue as the source of the cells is preferred due to its abundance and availability, and due to the fact that its removal should not adversely affect the patient being treated. Although less preferred, it is possible to obtain human perinephric fat from braindead but heart beating cadaver donors, or from donors other than the donor's surgery. The isolated endothelial cells are then deposited on a graft for implantation.

SUMMARY OF THE INVENTION

A device for collecting and processing tissue to produce an endothelial cell product is provided and includes a vessel for rinsing, draining, digesting and isolating tissue. The vessel has a rinsing and digesting chamber for containing tissue during processing. An inlet is in the rinsing and digesting chamber for connection in fluid communication with a liposuction means. A drain chamber is preferably provided in the vessel and is in fluid communication with the rinsing and digesting chamber. The drain chamber has a connection for fluid communication with a vacuum source. A screen is in the vessel for separating the rinsing and digesting chamber from the drain chamber to allow filtered transfer of rinsing solution from the rinsing and digesting chamber to the drain chamber.

An isolation chamber is in the vessel with the rinsing and digesting chamber and is separated from the rinsing and digesting chamber by another screen. A pair of ports are preferably separated by a channel each of which may be selectively attached in fluid communication with the channel by a valve means. Rinsed, digested, and processed tissue, produced under sterile conditions, may be collected within the channel as endothelial cell product. The vessel is generally cylindrical, having a frusto-conical end, to house the isolation chamber.

A preferred method of collecting and processing tissue to produce an endothelial cell product has the step of providing a collecting vessel for processing tissue to produce an endothelial cell product, the vessel having a rinsing and digesting chamber, a drain chamber and an isolation chamber. Another step is introducing tissue to be processed into the rinsing and digesting chamber. Another step is introducing rinsing solution into the rinsing and digesting chamber. A further step may be orienting the vessel to screen the tissue to be processed in the rinsing and digesting chamber from rinsing solution passed into the drain chamber. An additional step is introducing an enzyme into the rinsing and digesting chamber. This is followed by the steps of heating the tissue to be processed and the enzyme for a time period and at a temperature sufficient to digest, while agitating the rinsing and digesting chamber to digest the tissue with the enzyme. Centrifuging the vessel transfers the cells from the rinsing and digesting chamber into the isolation chamber. As a further step, the pellet of microvessel endothelial cells is isolated by selective operation of a valve means.

## DESCRIPTION OF THE DRAWINGS

Fig. 1 is a perspective view of a device illustrating the invention for collecting and processing tissue to produce an endothelial cell product;

Fig. 2 is a partial sectional view of the device of Fig. 1 taken along along lines 2-2 of Fig. 1, and showing the valve handles in a first position during centrifugation;

Fig. 2A is a schematic of the path of the flow through the valves and ports during centrifugation;

Fig. 3 is a partial sectional view of the device of Fig. 1 similar to that of Fig. 2, but with the valve handles in a second position after centrifugation;

Fig. 3A is a schematic of the possible paths of the flow through the valves and ports after centrifugation;

Fig. 4 is a side elevational view partially in section showing a device similar to that of Figs. 1, 2 and 3, but with a modified filter arrangement illustrating the invention;

Fig. 5 is a side elevational view partially in section showing a modification of the device of Fig. 4 and further illustrating the invention; and

Fig. 6 is a flow diagram illustrating the steps in the process of collecting and processing tissue to produce an endothelial cell product.

## DETAILED DESCRIPTION OF THE INVENTION

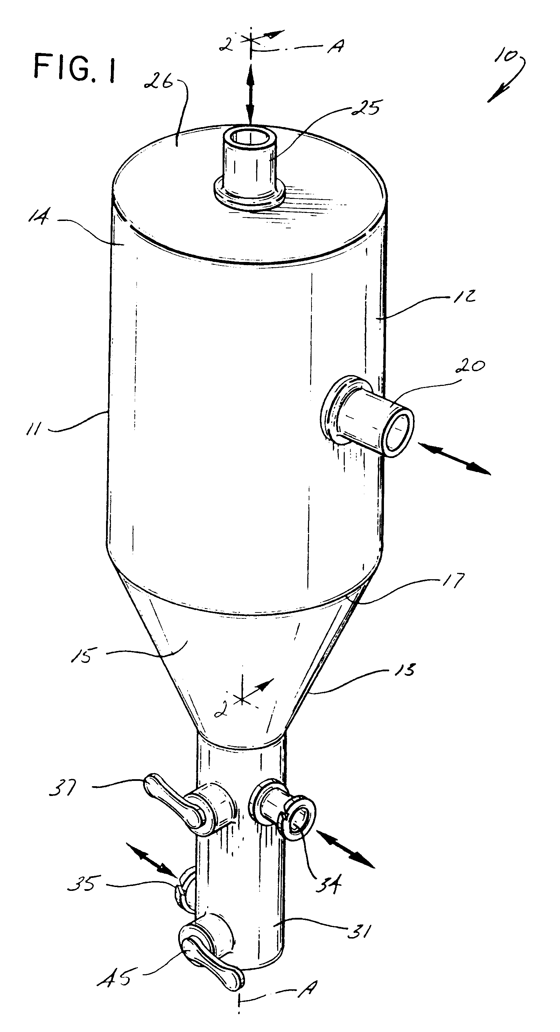

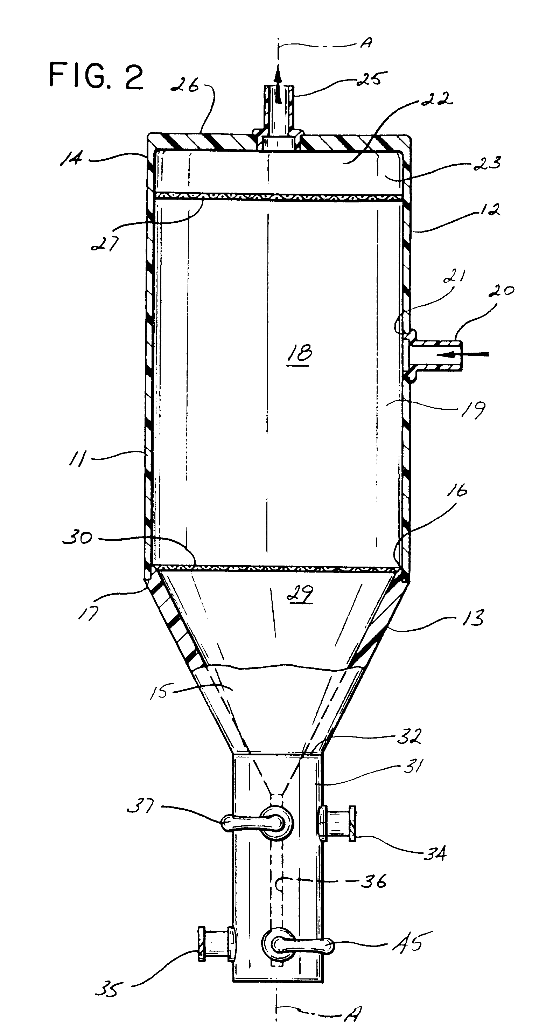

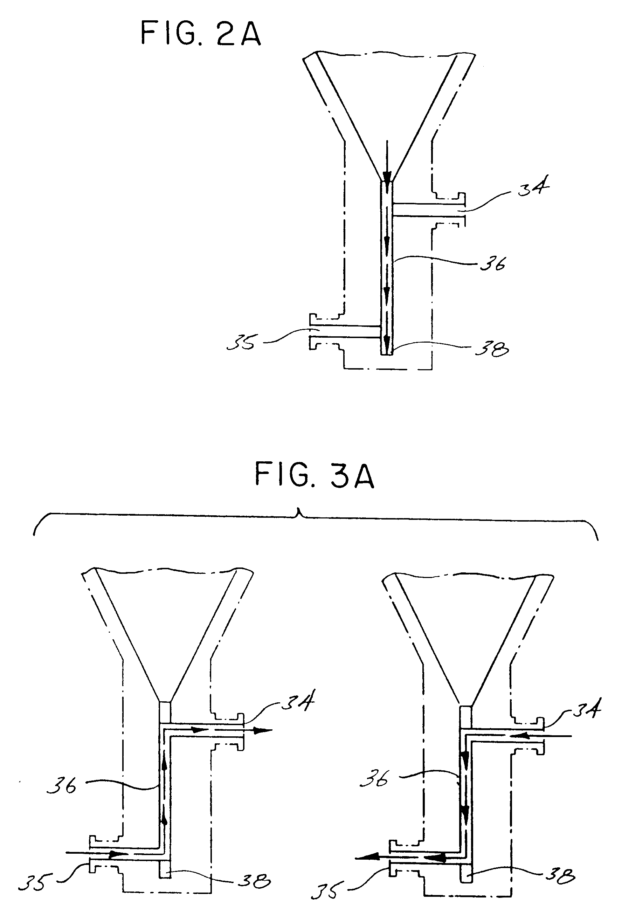

Referring to the drawings, in which like reference characters refer to like parts throughout the views thereof, Fig. 1 indicates generally the device 10 of the invention for collecting and processing tissue to produce an endothelial cell product. A vessel 11 is used for rinsing, draining, digesting and isolating tissue for the device 10. As shown in the side elevational views of Figs. 2-5, the vessel 11 is comprised of two parts, 12 and 13. While not required, vessel 11 can be made of a low cost polymer that is easy to shape and resistant to the substances that will be handled during processing endothelial cells. The upper part 12 is an inverted cup 14, and the lower part 13 is a frusto-conical funnel 15 with a top edge 16 that conjugates with an open bottom 17 of the inverted cup 14 to form an enclosure 18 in the vessel 11. A rinsing and digesting chamber 19 is contained within the inverted cup 14 of the vessel 11 so that tissue can be suitably handled during processing.

An inlet 20 is provided in side 21 of the rinsing and digesting chamber 19 for connection in fluid communication with an external liposuction means (not shown) for introduction of tissue having microvessel endothelial cells into the rinsing and digesting chamber 19. As shown in Fig. 2, drain chamber 22 is located within an upper inside portion 23 of the inverted cup 14 of the vessel 11 and is in fluid communication with the rinsing and digesting chamber 19.

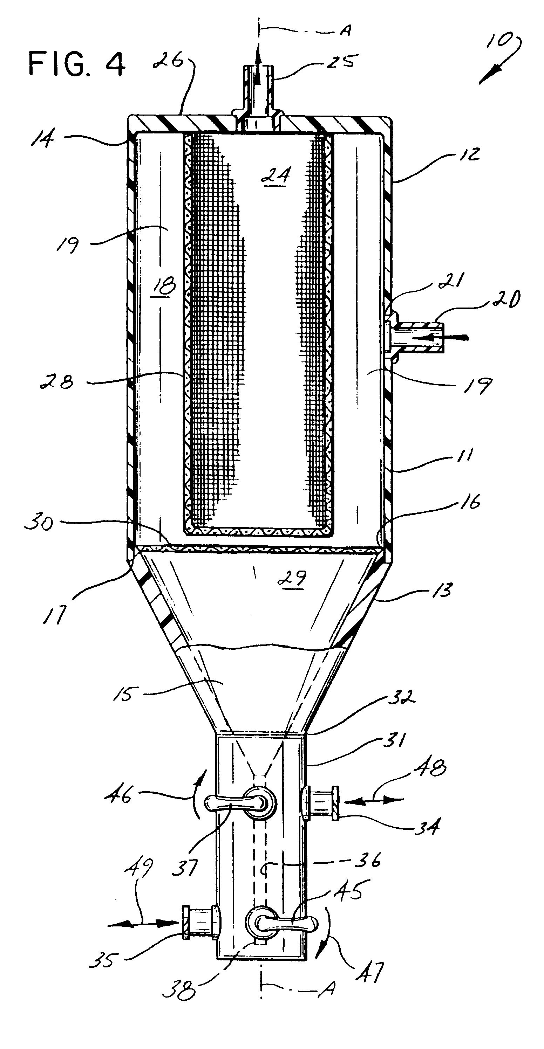

In Fig. 4, an alternate drain chamber 24 is centrally located within enclosure 18. Both drain chambers 22 and 24 have a connection 25 located to pass through a top 26 of the inverted cup 14 for allowing fluid communication with a vacuum source (not shown) applied thereto.

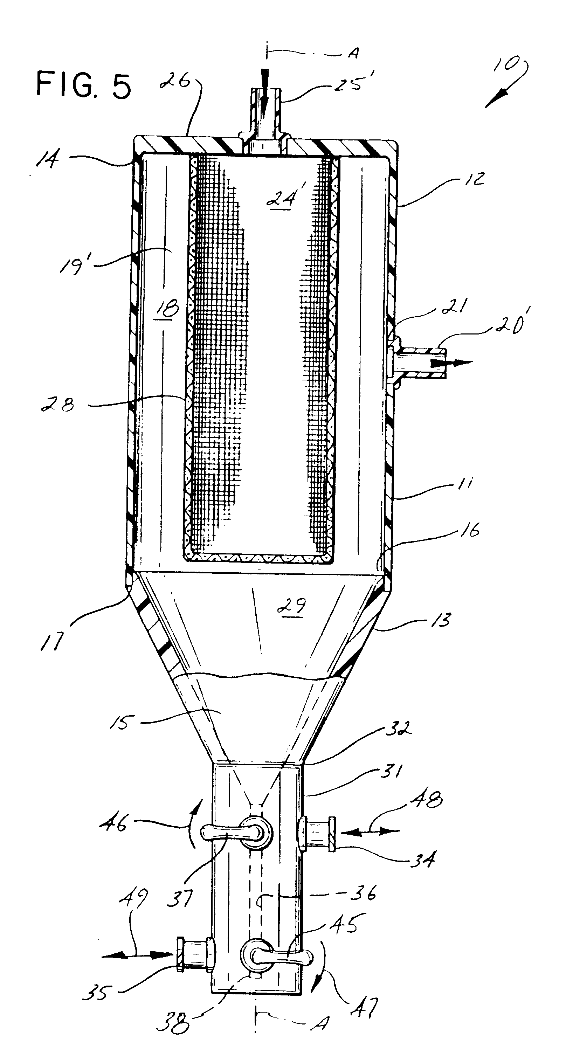

In Fig. 5, the device is similar to that of Figs. 2, 3 and 4, but the connections of 20 and 25 are reversed. Specifically, the inlet in Fig. 5 is 25' and the outlet connection is 20', since the drain chamber is now 19' and the rinsing and digesting chamber is 24'. The rinsing and digesting chamber 24' is centrally located within enclosure 18.

In Fig. 2, an upper screen 27 positioned within vessel 11, inside inverted cup 14, separates the rinsing and digesting chamber 19 from the drain chamber 22 to allow filtered transfer of rinsing solution from the rinsing and digesting chamber 19 to the drain chamber 22. The screen 27 may be of various configurations. As shown in Fig. 2, the screen 27 is flat, and separates the rinsing and digesting chamber 19 from the drain chamber 22. As a result, the drain chamber 22 is adjacent to upper inside portion 23 of the inverted cup 14 and the rinsing and digesting chamber 19 is immediately therebeneath. In Fig. 4 the screen 28 is shown in the shape of a cylinder, which defines the drain chamber 24 within the center of the inverted cup 14 along an axis A thereof. The rinsing and digesting chamber 19 circumscribes or fits about in surrounding fashion the screened in drain chamber 24.

Lower part 13 defines a frusto-conical funnel 15 with an internal chamber 29 and having a top edge 16 which is connected to the open bottom 17 of inverted cup 14 to form a combined enclosure 18, 29. Referring to the embodiments of Figs. 2, 3 and 4, each has a flat transverse screen 30. Screen 30 is positioned adjacent top edge 16 of funnel 15. The embodiment of Fig. 5 does not include screen 30.

A tube 31 connects to apex 32 of funnel 15. Tube 31 carries a pair of spaced apart ports 34, 35, connected to a vertical channel 36, positioned centrally of tube 31. Ports 34, 35 are for selective communication with channel 36.

Selective communication with ports 34, 35 is accomplished by valves 37, 45, respectively, as shown in Fig. 4, so as to connect ports 34, 35 with channel 36 selectively. To achieve this, valve 37 is rotated as indicated by arrow 46, and valve 45 is rotated as indicated by arrow 47. Arrows 48, 49 show flow direction two ways through ports 34, 35.

The selective fluid communication is accomplished by valve means 37 operatively associated with the pair of ports 34 and 35 and located at each end of the channel 36 for selectively connecting ports 34 and 35 in fluid communication with channel 36. Using valves 37, 45 permits rinsed, digested and processed tissue produced under sterile conditions to be isolated within the vessel as a microvessel endothelial cell pellet. To remove the pellet, the valves 37, 45 are adjusted to allow the pellet to be drawn off through one of ports 34 or 35.

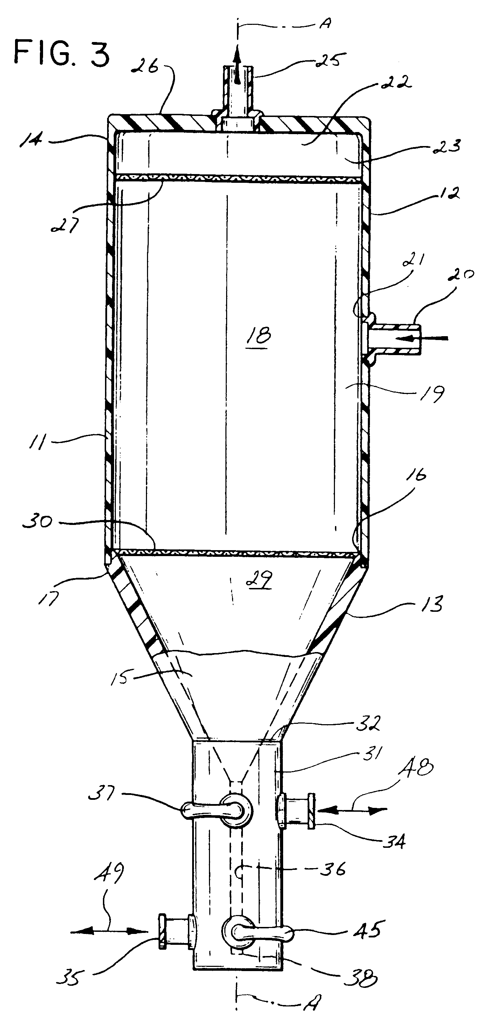

Fig. 2A shows a schematic view of the flow path through valves 37, 45 during centrifugation. This flow path traps red blood cells in a trap 38 at the bottom of channel 36 as shown in Figs. 2, 3, 4 and 5. Trap 38 collects red blood cells which are denser than the microvessel endothelial cells in the pellet. After centrifugation, the valves 37, 45 can be adjusted as shown schematically in Fig. 3A. Two possible flow paths are shown such that the isolated cells can be removed from channel 36 and trap 38 by vacuum or pressure applied to either ports 34 or 35. The valves 37, 45 may be simple stopcocks as are commonly found in medical accessories. The channel 36 is shaped to contain therein the pellet of microvessel endothelial cells (not shown).

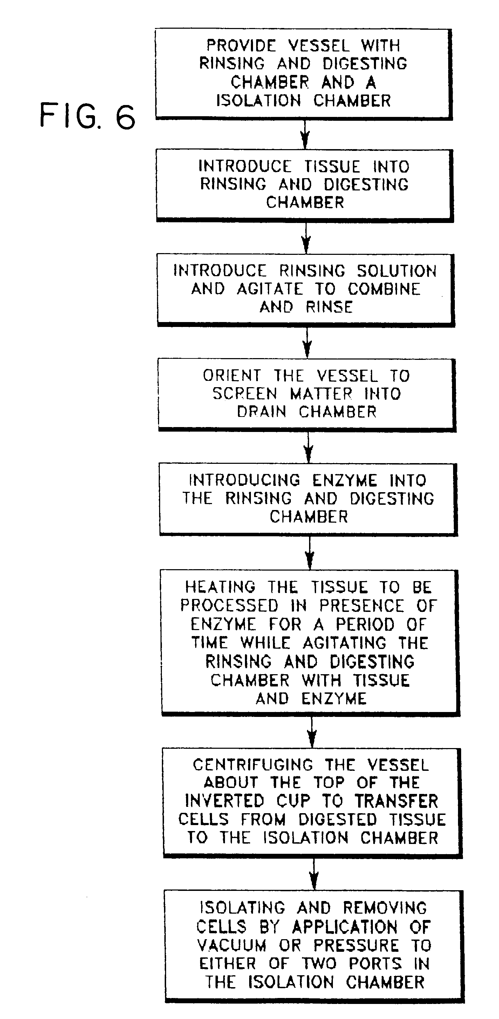

A method of collecting and processing tissue to produce an endothelial cell product has various steps shown on the schematic diagram of Fig. 6, as blocks. To perform the method, the collecting vessel for processing tissue to produce an endothelial cell product is provided. Vessel 11 has the rinsing and digesting chamber 19, the drain chamber and the isolation chamber.

Introducing tissue to be processed into the rinsing and digesting chamber 19 is a preliminary step of the processing method. The tissue may be obtained from a liposuction procedure. Orienting the vessel permits vacuum and gravity to filter off unneeded liquid from the tissue sample through a screen, which separates the tissue to be processed in the rinsing and digesting chamber 19 from rinsing solution which passes into the drain chamber 22 or 24. The method is continued by introducing an enzyme into the rinsing and digesting chamber 19, and heating the tissue to be processed in the presence of an enzyme for a period of time, and at a temperature sufficient to digest the tissue. The digestion is assisted by the step of agitating the rinsing and digesting chamber 19 containing the tissue and enzyme.

Centrifuging the vessel about the top 26 of the inverted cup 14 collects the cells from the digested tissue from the rinsing and digesting chamber 19 in the isolation chamber 29. Any red blood cells in the rinsed and digested tissue will be collected in the trap 38 at the bottom of channel 36 because, as mentioned, the red blood cells are denser and heavier than the microvessel endothelial cells. The method has the further step of isolating the digested tissue in the form of a pellet of microvessel endothelial cells by selective application of the valves 37, 45, as discussed above.

While the forms of apparatus and the method described herein constitute preferred embodiments of the invention, it is to be understood that the invention is not limited to these preferred forms of methods and apparatus, and that changes can be made therein without departing from the scope of the invention which is defined in the appended claims. For example, various screens with pore configurations may be developed to enhance separation. Moreover, centrifugal force control and agitation intensity may be varied to enhance output quantity and quality of the final cell pellet. In addition, the main digestion chamber, while shown in cylindrical form in cross-section, may be configured to be square for certain operating room accommodations.

## Claims

1. A device for collecting and processing tissue to produce an endothelial cell product comprising:
   a vessel having a rinsing and digesting chamber, a drain chamber, and an isolation chamber which chambers allow the rinsing, digestion and processing of tissue to produce within the vessel under sterile conditions endothelial cell product.

2. The device of Claim 1 wherein the rinsing and digesting chamber is separated from the drain chamber by a screen.

3. The device of Claim 2 wherein the rinsing and digesting chamber is separated from the isolation chamber by a screen.

4. The device of Claim 2 wherein said screen separates said rinsing and digesting chamber from said drain chamber and said isolation chamber, said drain chamber surrounds said rinsing and digesting chamber; and

said drain chamber has a connection to a vacuum source.

5. The device of Claim 4 wherein the rinsing and digesting chamber includes an inlet for connection in fluid communication with a source of fat; said connection being on the side of said screen opposite said drain chamber.

6. The device of Claim 1 wherein the isolation chamber has a pair of ports connected by a channel, and valve means between the channel and each of the pair of ports to selectively control flow to and from said channel through each of the pair of ports.

7. The device of Claim 6 wherein the channel has a trap at the bottom thereof and the channel is shaped to collect therein a pellet of microvessel. endothelial cells and segregate red blood cells in the trap by centrifugation.

8. The device of Claim 1 wherein the vessel defines a cylindrical chamber with a frusto-conical end for housing the isolation chamber, and including a screen within the chamber for separating the rinsing and digesting chamber from the drain chamber.

9. A method for collecting and processing tissue for producing an endothelial cell product comprising:

providing a collecting vessel for processing tissue to produce an endothelial cell product, the vessel having a rinsing and digesting chamber, a drain chamber and an isolation chamber;

introducing tissue to be processed into the rinsing and digesting chamber;

introducing rinsing solution into the rinsing and digesting chamber;

orienting the vessel to screen the tissue to be processed in the rinsing and digesting chamber from rinsing solution passed into the drain chamber;

introducing an enzyme into the rinsing and digesting chamber;

heating the tissue to be processed and the enzyme for a period of time and at a temperature sufficient to digest while agitating the rinsing and digesting chamber to digest the tissue with the enzyme,

centrifuging the vessel to transfer the cells from the digested tissue from the rinsing and digesting chamber into the isolation chamber, and

isolating the cells from the digested tissue in a channel as a pellet of microvessel endothelial cells.

10. The method of Claim 9 with the further step of isolating the cells from the digested tissue in a channel as a pellet of microvessel endothelial cells by application of a valve means.

FIG. 1

FIG. 2

# FIG. 2A

# FIG. 3A

FIG. 3

FIG. 4

FIG. 5

FIG. 6

```
┌─────────────────────────┐
│     PROVIDE VESSEL WITH  │
│    RINSING AND DIGESTING │
│       CHAMBER AND A      │
│     ISOLATION CHAMBER    │
└─────────────────────────┘
             │
             ▼
┌─────────────────────────┐
│    INTRODUCE TISSUE INTO │
│    RINSING AND DIGESTING │
│          CHAMBER         │
└─────────────────────────┘
             │
             ▼
┌─────────────────────────┐
│  INTRODUCE RINSING SOLUTION │
│   AND AGITATE TO COMBINE │
│         AND RINSE        │
└─────────────────────────┘
             │
             ▼
┌─────────────────────────┐
│    ORIENT THE VESSEL TO  │
│    SCREEN MATTER INTO    │
│       DRAIN CHAMBER      │
└─────────────────────────┘
             │
             ▼
┌─────────────────────────┐
│   INTRODUCING ENZYME INTO │
│   THE RINSING AND DIGESTING │
│          CHAMBER         │
└─────────────────────────┘
             │
             ▼
┌─────────────────────────┐
│   HEATING THE TISSUE TO BE │
│   PROCESSED IN PRESENCE OF │
│   ENZYME FOR A PERIOD OF │
│   TIME WHILE AGITATING THE │
│    RINSING AND DIGESTING │
│     CHAMBER WITH TISSUE  │
│         AND ENZYME       │
└─────────────────────────┘
             │
             ▼
┌─────────────────────────┐
│   CENTRIFUGING THE VESSEL │
│     ABOUT THE TOP OF THE │
│   INVERTED CUP TO TRANSFER │
│  CELLS FROM DIGESTED TISSUE │
│   TO THE ISOLATION CHAMBER │
└─────────────────────────┘
             │
             ▼
┌─────────────────────────┐
│   ISOLATING AND REMOVING │
│   CELLS BY APPLICATION OF │
│   VACUUM OR PRESSURE TO  │
│   EITHER OF TWO PORTS IN │
│     THE ISOLATION CHAMBER │
└─────────────────────────┘
```